(19) Europäisches Patentamt
European Patent Office
Office européen des brevets

(11) **EP 3 674 703 A1**

(12) **EUROPEAN PATENT APPLICATION**

(43) Date of publication:
**01.07.2020 Bulletin 2020/27**

(51) Int Cl.:
**G01N 33/00** (2006.01)      **G01N 22/04** (2006.01)
**A01G 25/16** (2006.01)

(21) Application number: **19166018.2**

(22) Date of filing: **28.03.2019**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **31.12.2018 PT 2018115239**

(71) Applicant: **INESC TEC - Instituto de Engenharia de
Sistemas e
Computadores, Tecnologia e Ciência
4200-465 Porto (PT)**

(72) Inventors:
• **DUARTE DOS SANTOS, Manuel Cândido**
**4200-465 Porto (PT)**
• **BATISTA NEVES DOS SANTOS, Filipe**
**4200-465 Porto (PT)**
• **GOMES BORGES, Rodrigo**
**4200-465 Porto (PT)**

(74) Representative: **Patentree
Edificio Net
Rua de Salazares, 842
4149-002 Porto (PT)**

(54) **METHOD AND DEVICE FOR MEASURING WATER PRESENT IN VEGETATION**

(57)   Device, and operation method thereof, for measuring plant water present in vegetation comprising: an microwave emitter of a displaceable beam; a microwave receiver; an electronic data processor connected to receive a signal from the microwave receiver; wherein the microwave emitter is arranged to direct said displaceable beam towards the microwave receiver for measuring attenuation of the beam passing through each of a plurality of locations of the vegetation; wherein the electronic data processor is configured for calculating the attenuation of the displaceable beam between the emitter and receiver to obtain a 2D image corresponding to the measurement of water present in the vegetation. In particular, said beam is displaceable by the device being mounted on a vehicle which is movable along the vegetation to be measured in a horizontal direction, the microwave emitter and microwave receiver being jointly mounted in opposite ends of an arched structure.

Fig. 5

## Description

## TECHNICAL FIELD

The present disclosure relates to a device and method for measuring water present in vegetation using radio frequency attenuation.

## BACKGROUND

Water plays a central role in farming processes and this input must be tightly controlled and corrective and precise actions must be taken to support healthy crops. Thus, precision agriculture together with agricultural robotics has been exploiting the use of variable rate technologies (VRT) for controlling the rate of agronomic inputs (e.g. water) based on contextual features such as plant status and location.

A traditional VRT system only uses information provided by agro data cloud systems to determine the amount of agronomic input required to be applied. However, these systems do not get real-time feedback about the real requirements of the plant needs (e.g. water) and the real amount of products applied.

Real-time and remote solutions (using satellites or aerial vehicles) disclosed in prior art comprise mostly radar scatterometers or optical-based systems.

Radar scatterometers namely Synthetic Aperture Radars (SAR), can monitor vegetation phenology, using an imaging-based system that transmits microwaves and receives the reflections of the backscattered signals. Although satellite SAR systems are specialized in providing average water content of wide planting regions they do not have resolution to measuring water content at a single plant or even at leave-by-leave-scale (i.e. precision agriculture applications). Moreover, satellite SAR systems are strongly affected by the interference of other elements during the scattering processes such as the soil moisture, the surface type, the terrain topology, the techniques used during the cultivation process and the atmospheric transmission characteristics [2].

Optical-based methods can be used for water sensing by analyzing the reflected electromagnetic signal by vegetation canopies. This reflectance is largely affected by the biological properties of the plant, and in the regions of 0.7 to 1.4 $\mu$m (near-infrared wavelengths) and 1.3 to 2.5 $\mu$m (middle-infrared wavelengths) of the electromagnetic spectrum, the reflectance is affected by the quantity of water in the leaves [1]. However, the accuracy of optical-based methods is negatively affected by atmospheric conditions. Moreover, water sensing using optical-based methods is limited to the contribution of the superficial layers of the plants because only reflected information is used.

Document KR101824652 relates to a device for calculating the moisture content of a plant. The device for calculating the weight and moisture content of a plant includes a cultivating bed on which a medium is mounted, wherein nutrient solution is supplied to enable the plant to be grown, a weight measuring portion, and a control portion. The weight measuring portion is configured to measure the weight of the medium mounted on the cultivating bed by floating the cultivating bed in the air. The control portion is configured to collect a measurement value from the weight measuring portion and calculate the weight and the water content of the plant by using the collected measurement value.

The prior art does not provide feasible practical solutions of measuring water content at a plant scale that can be adapted to different atmospheric and terrain conditions.

References

[1] E.Raymond Hunt and Barrett N Rock. Detection of changes in leaf water content using near- and middle-infrared reflectances. Remote Sensing of Environment, 30(1):43 - 54, 1989.

[2] G. Schiavon, D. Solimini, and A. Burini. Sensitivity of multi-temporal high resolution polarimetric C and L-band SAR to grapes in vineyards. In 2007 IEEE International Geoscience and Remote Sensing Symposium, pages 3651-3654, July 2007.

These facts are disclosed in order to illustrate the technical problem addressed by the present disclosure.

## GENERAL DESCRIPTION

This document discloses a method and device for measuring the water content in plants using radio frequency attenuation for real-time water sensing. The method comprises according to an embodiment transmitting electromagnetic radiation with frequencies in particular above 20 GHz on one side of a plant, receive the transmitted signal on the other side of the said plant, and analyze the attenuation of the signal caused by the water content of the plant being irradiated.

The implemented approach includes analyzing the attenuation caused by leaves, plants, and elements containing water over a transmission between a pair of high gain antennas.

The presented method and device are immune to dry elements that obstruct the view or to illuminations changes, which would impair the measurement by an optical based system.

In an embodiment the system can be used in combination with a spraying system, analysing the plants right before each spraying passage and right after the said passage, giving a real-time accurate feedback of the amount of product sprayed.

In an embodiment of an anti-drift panel/drift recovery-based sprayer the system can monitor the water quantity right before the product application and monitor water quantity application on leaf and plant surfaces, without need to be in contact with the plants (as the optical based systems require).

The antenna array system can be attached to any place of mobile machinery (autonomous or non-autonomous) and/or in machinery implemented in such way that the plants will be in the middle of antenna array system. To avoid the collision between antenna array system and the plants an active pan and tilt system may be used to control the antenna array system posture.

It is disclosed a device for measuring plant water present in vegetation comprising:

a microwave emitter of a displaceable beam;
a microwave receiver;
an electronic data processor connected to receive a signal from the microwave receiver;
wherein the microwave emitter is arranged to direct said displaceable beam towards the microwave receiver for measuring attenuation of the beam passing through each of a plurality of locations of the vegetation;
wherein the electronic data processor is configured for calculating the attenuation of the displaceable beam between the emitter and receiver to obtain a 2D image corresponding to the measurement of water present in the vegetation.

In an embodiment, the microwave receiver comprises a plurality of individual receivers.

In an embodiment, the microwave receiver comprises a plurality of individual microwave receivers linearly arranged and distributed in particular along a vertical direction.

In an embodiment, the microwave emitter is arranged to direct said displaceable beam towards each of said individual microwave receivers.

In an embodiment, the microwave emitter is arranged to direct said displaceable beam towards one by one of said individual microwave receivers;

In an embodiment, the microwave emitter comprises a steerable beam antenna, in particular a steerable beam phase-shift antenna, for directing said microwave beam between each of said individual microwave receivers.

In an embodiment, the microwave emitter comprises a plurality of individual emitters which are individually switchable for directing said microwave beam between each of said individual microwave receivers.

In an embodiment, the microwave emitter comprises a plurality of individual microwave emitter linearly arranged and distributed in particular along a vertical direction.

In an embodiment, said beam is displaceable by the device being movable along the vegetation to be measured in particular along a horizontal direction, in particular the microwave emitter and microwave receiver being jointly mounted in the device, further in particular the microwave emitter and microwave receiver being jointly mounted in opposite ends of an arched structure such that the vegetation can pass below the arch between the emitter and the receiver.

In an embodiment, the device is a vehicle and the microwave emitter and microwave receiver are mounted on the vehicle for moving along the vegetation.

An embodiment comprises a satellite geolocation sensor, a radiofrequency geolocation sensor or a displacement sensor for synchronizing the measurement of water present in the vegetation with the device location.

An embodiment comprises a second microwave emitter and a second microwave receiver mounted on the vehicle for moving along the vegetation, such that the second emitter-receiver pair measure attenuation of the beam passing through each of a plurality of locations of the vegetation after the first emitter-receiver pair have measured attenuation of the beam passing through each of the same plurality of locations of the vegetation.

In an embodiment, the electronic data processor is configured for calculating the difference in attenuation of the displaceable beam between the first emitter-receiver pair and the second emitter-receiver pair to obtain a 2D image corresponding to the difference in the measurement of water present in the vegetation.

An embodiment comprises a dispenser for applying the treatment and being arranged to:

measure the water present in the vegetation before applying the treatment and adjust the amount of treatment applied in respect of the measured water; and/or
measure the water present in the vegetation before and after applying the treatment for calculating the difference in the measurement of water present in the vegetation to measure the applied treatment.

It is also disclosed a method of operation of a device for measuring plant water present in vegetation, said device comprising a microwave emitter of a displaceable beam; a microwave receiver; an electronic data processor connected to receive a signal from the microwave receiver; said method comprising:

directing said displaceable beam by the microwave emitter towards the microwave receiver for measuring attenuation of the beam passing through each of a plurality of locations of the vegetation;
calculating, by the electronic data processor, the attenuation of the displaceable beam between the emitter and receiver to obtain a 2D image corresponding to the measurement of water present in the vegetation.

## BRIEF DESCRIPTION OF THE DRAWINGS

The following figures provide preferred embodiments for illustrating the disclosure and should not be seen as limiting the scope of invention.

**Figure 1:** Schematic representation of an overview of an embodiment of the disclosed device.

**Figure 2A and Figure 2B:** Schematic representations of views of possible embodiments using multiple emitting and receiving elements and their direct radiation pathways

**Figure 3:** Schematic representation of a diagram of an embodiment of the antenna array with switches.

**Figure 4:** Schematic representation of a view of an embodiment of antenna arrays with phase shifters.

**Figure 5:** Schematic representation of a view of an embodiment of the disclosed device combined with a vehicle and a spraying system for agriculture applications.

**Figure 6:** Comparison between the real image of the cactus-like plant and the resulting image after the analysis of the collected data obtained according to an embodiment of the disclosure.

**Figure 7:** Leaf with a dried region (bottom) and the respective result of the test scan obtained according to an embodiment of the disclosure.

**Figure 8A, Figure 8B, and Figure 8C:** Images of the analysis of a green leaf (A), the analysis of the same leaf with water on its surface (B) and the difference between the two images (C) obtained according to an embodiment of the disclosure.

**Figure 9A, Figure 9B, and Figure 9C:** Images of a green leaf before (A) and after been sprayed with water (B) and the difference between the two images (C) obtained according to an embodiment of the disclosure.

## DETAILED DESCRIPTION

This document discloses a method and device for remote water sensing using transmitted microwaves, and more particularly for measuring water content in a plant. Unlike the optical-based methods that measure and analyse the visible and infrared signals reflected by the object of interest, the method and device of this document uses transmitted microwaves enabling sensing deeper plant layers and being immune to the interference caused by illumination changes or by the presence of dry elements on the accuracy of the predicted water content values.

The water content measurement can be used to characterize the water status of plants or as an input to the control other devices and systems (e.g. spraying system), adjusting automatically the amount of the agronomic inputs to be sprayed. The disclosed device and method is capable to create a 2D or 2.5D, or even three-dimensional, map of water content present in the plant or group of plants.

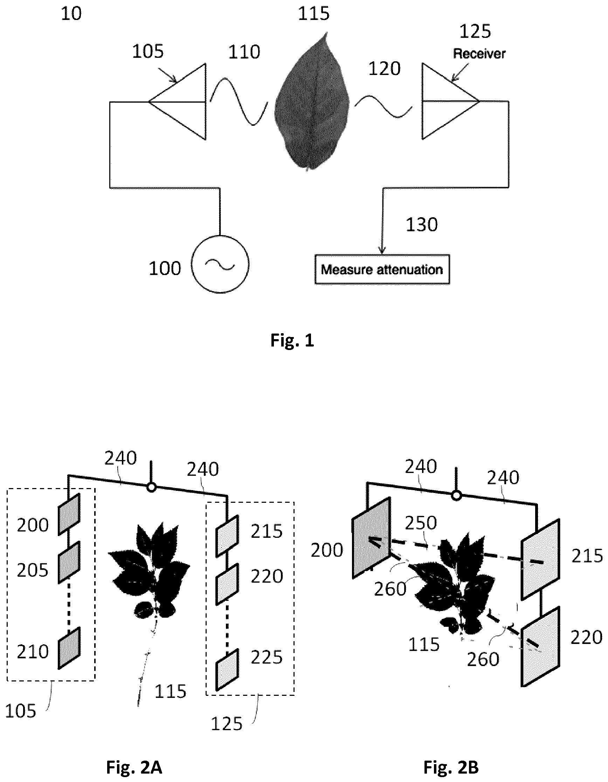

Reference is made to **Fig. 1** which is a schematic view of the device and illustrates the method to measure water content in plants. As depicted by **Fig. 1,** the device **10** comprises an emitter **105** that generates an electromagnetic radiation **110** (e.g. microwaves) towards a plant or a region of the plant (e.g. leaves) **115.**

The generated electromagnetic radiation **110** may be, but is not limited to, frequencies around 20 GHz, due to the effects of water presence in the signal radiation path, which is established between an emitter **105** and a receiver **125.** In fact, the receiver measures a peak of attenuation around 20 GHz when water (gas or liquid form) or a water containing object is between the receiver and the emitter antennas (direct radiation path). The said electromagnetic radiation **110** is transmitted but attenuated along the plant **115.** The transmitted electromagnetic radiation **120** is sensed by the receiver **125** and the signal attenuation is measured by the unit **130**. The attenuation caused by the plant **115** leads to a loss in power of the said generated electromagnetic radiation **110,** depending on the water content of the plant **115.**

In free space conditions meaning absence of any object or plant 115 in the direct radiation path between the emitter and the receiver, the attenuation in the radiated signal is given by free space path loss ($L_{fs}$) calculated by the **Equation 1,** where $f$ is the frequency, $c$ is the speed of light in the vacuum and $D$ is the distance between the emitter and the receiver.

$$L_{fs} = 20\log_{10}\left(\frac{4\pi f D}{c}\right) \text{ (dB)} \quad \textbf{(Equation 1)}$$

In presence of a water-containing object (e.g. **plant)** the measured signal loss between the emitter and the receiver is significantly increased when compared to the signal loss value obtained in the calibration, which can be performed in free space condition ($L_{fs}$) or in a real environment (humid air) without a plant in the direct radiation path.

The plant water content is predicted by the differential amount of signal loss (attenuation) between the plant measure and the calibration.

Frequencies in the range of 20GHz were applied in this example because they can go through dried plants without suffering signal loss so not changing $L_{fs}$ value.

Higher frequencies than 20 GHz may be applied to improve water sensing sensitivity. However, higher frequencies lead to increased free space power loss what requires decreasing the distance between the emitter **105** and the receiver **125** for the same radiated power. Improved spatial resolution may be achieved by the use of higher frequencies than 20GHz. Other different frequencies may be selected and used to avoid excessive losses due to specific use cases or to avoid reflections occurring at a specific frequency. Also, different frequencies may be used simultaneously, in order to electronically or computationally select the frequency signal or signals providing the best measurement.

In an alternative embodiment power reflection may be also used to predict the plant water content. However, the power reflected by the said plant is extremely low which leads to decreased sensitivity comparing to the disclosed method using attenuation between the emitter and the receiver.

In order to water sensing different plants with different shapes and sizes, a variable number of emitters and receivers can be used as disclosed in **Fig.2A.** In this embodiment, the emitter element **105** may be an array comprising at least an emitter antenna **200** and the receiver element **125** may be an array with at least a receiver antenna **215.** The term emitter/receiver pair will be used to describe any combination of an emitter from a multitude of emitters in an array and a receiver from a multitude of receivers in an array that can establish a link.

The said emitter array **105** and the said receiver array **125** are connected by a structure **240** (e.g. metallic bar) to control the distance between them, i.e. the direct radiation path length. The said structure **240** may be connected to a mobile structure (e.g. robotic arm) that can move the said emitter and receiver arrays to measure the water content in different points of the plant **115.**

Reference is made to **Fig. 2B** to explain the different radiation paths that characterize each emitter/receiver pair. For example, the radiation path **250** characterize the link between the emitter **200** and the receiver **215** while the radiation path **260** characterize the link between the emitter **200** and the receiver **220**. Therefore, the same emitter can provide different radiation paths which can be sensed by different receivers. This increase in the number of direct radiation paths can be used to improve the spatial resolution and the accuracy of the measurement because each direct radiation path between an emitter/receiver pair can be used to measure an attenuation value, and thus water content, that can be mapped. This map can be presented as a water content image.

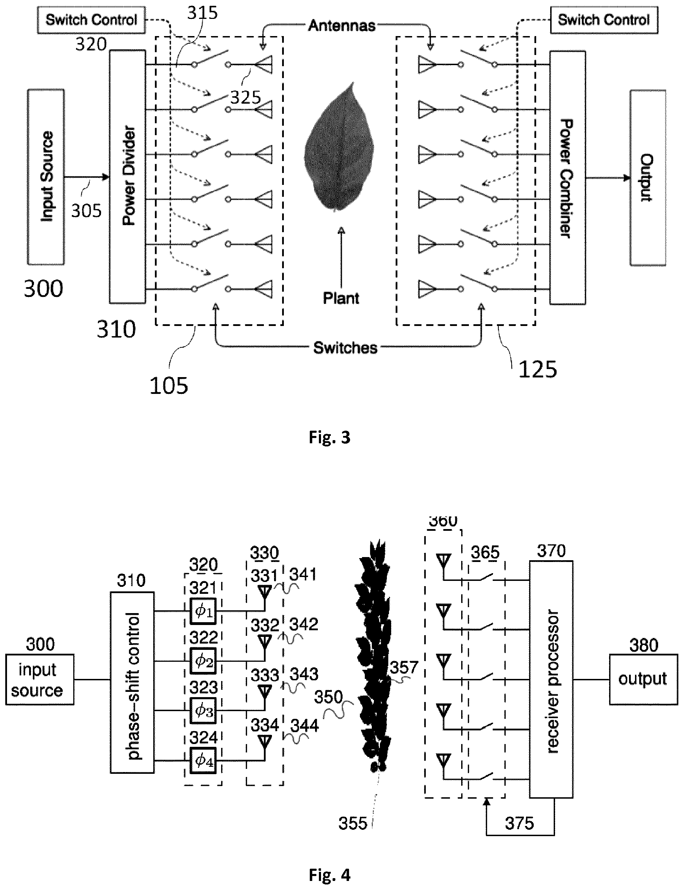

To mitigate the interference caused by reflections on the water sensing, this disclosure implements isolation between signals paths by controlling along time the operation of non-simultaneous emitter/receiver pairs. Reference is made to **Fig. 3** to explain how the emitter/receiver pairs can be established.

References is made to **Fig. 3** to disclose the electric-scan based in switch controlling. An input source **300** generates the input signal **305** which is injected in a power divider **310**. This power divider **310** is composed of as many power channels **315** as the number of emitting antennas. Each power channel **315** is connected to a switch control **320** that controls the power delivered to each emitting antenna **325.**

Each emitter/receiver pair is activated at each given moment, with the intensity of the emitted signal being attenuated by the objects and plants in the signal path between the emitter/receiver pair. In this embodiment, the electrical scan consists of controlling the switches connected to each antenna as shown in Fig. 3 turning on and off the different emitter/receiver pairs in the arrays to scan the whole area.

Reference is made to **Fig. 4** to describe a preferable embodiment of the disclosure disclosed herein comprising electric-scan based in phase shifting for beam steering control. An input source **400** generates the input signal which is injected in a phase-shift control **410.** This phase-shift control **410** is composed of as many power channels as the number of emitting antennas. Each power channel supplies a phase shifter **421-424.** A phase control module **410** individually controls each phase shifter **421-424.** Each phase shifter **421-424** is connected to an emitting antenna **431-434** that emits an emitted signal **441-444.** At least two phase shifters **421** and **422** and correspondent two emitting antennas **431** and **432** are required for steering the emitted signals **441-442** in to an emitted beam **450.**

This electronically steerable array of antennas enables the control of the direction of the emitted beam **450** (beam). In this configuration, all the emitting antennas of the array **450** emit at the same time. The combination of the individual emitted signals of each antenna of the array creates an emitted beam, directing the radiation pattern of the array in a specific direction.

The emitted beam angle control is achieved by shifting the phase of the signal emitted from each emitting antenna to provide constructive or destructive interference so as to steer the emitted beam **450** in the desired direction. The direction of the emitted beam **450** is therefore changed by controlling the phase shifters **420** connected to each radiating elements of the array **430.**

The beam steering of the array disclosed in the **Fig. 4** is performed by controlling the phase of the signal for each antenna. The direction of the beam is defined by the phase difference between each consecutive element of the array. This phase difference ($\Delta\varphi$) is obtained using the **Equation 2,** where d is the predetermined distance between the radiating elements, $\lambda$ is the wavelength (e.g. 20GHz) and $\theta_s$ is the desired angle of the beam.

$$\Delta\varphi = \frac{360^o \times d \times sin(\theta_s)}{\lambda} \qquad \text{(Equation 2)}$$

The emitted beam **450** suffers attenuation while it is transmitted along the plant **455,** continuing as transmitted beam **457** towards the receiving elements **460.** Each receiving element is connected to a switch **465,** which is controlled by switch control **475,** turning on and off individual switches from **465.** The receiver processor **470** determines the water content value, based in measured the attenuation values, to each element of the map. At each time the receiver processor **470** has access to the present emitted beam angle which can be used to weight or correct the map of attenuations and consequent water content values that are outputted **480.**

The determined attenuation (power loss) values are normalized to the range of 0 to 1 using the maximum and minimum values of the matrix with the absolute values. A grayscale image of the object, plant or leaf is therefore generated where the brightest part of the image represents the area with the greatest attenuation and, therefore, a greater water quantity, whereas, the darkest areas of the image represents a lower signal attenuation corresponding to areas with less water content.

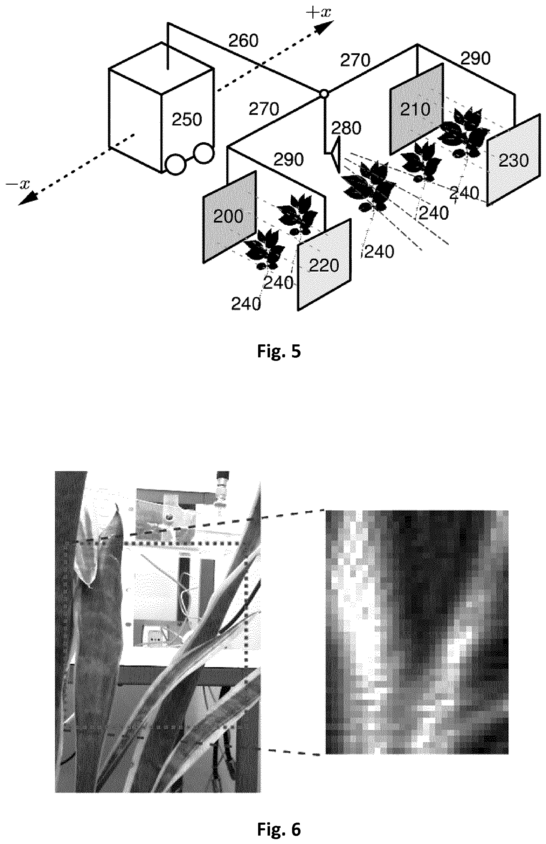

Reference is made to **Fig. 5** to disclose a preferential embodiment of the present disclosure for agriculture applications. In this embodiment a vehicle **550** is connected through an element **560** to an apparatus to automatically spray **580** an agronomical product. In this embodiment, and assuming that the said vehicle **550** is moving along the positive x axis, the plant **540** water content is determined by the device that comprises the emitter/receiver pair **510** and **530,** connected to a fixed structure **590** to ensure a constant distance, prior to the spray step. A second emitter/receiver pair **500** and **520** is used to provide a second water content measurement that is carried out after the spraying step. The elements **500** and **520** are arranged in face-to-face manner where the plant **540** is between them. The emitter/receiver pair are attached to the sprayer element **580** through an element **570.** The plant water content value determined by the pair **510** and **530** can be used to control the amount of agronomical product sprayed by the element **580.** The vegetation elements **540** are sprayed using a sprayer **580** that is attached to a vehicle **550** that moves in parallel with the alignment of the vegetation (x axis).

Based on the differences of attenuation (water content) sensed by the two said emitter/receiver pairs **(510, 530** and **500, 520)** the amount of sprayed agronomical product is determined as well as its position and scattering on the plant considering the data collected during the scanning step (beam steering). In case of low product distribution, the vehicle **550** moves backwards, repeating the spraying step. Each transmitter transmits a signal with transmit power and known frequencies. The positioning of the vehicle is known over time. Along the +/- x offset each receiver that records the attenuation over time. According to the direction of travel of the vehicle **550,** one receiver will record the power of the received signal before spraying and the other the power of the signal after spraying. The difference in power attenuation received, after correction of distance between receivers, allows to determine the quality of spraying.

The longitudinal velocity of the vehicle can be changed in order to increase or decrease the longitudinal map detail as data collection is synchronised with the displacement of the vehicle, for example by satellite or radiofrequency geolocation, or for example by a rotational encoder placed at the vehicle wheels or transmission.

The signal attenuation that has a direct relation to the water quantity is integrated with the system geolocation to build a three-dimensional map where each observed spot, defined in a North-East-Up referential coordinate system. This three-dimensional signal attenuation map is after converted to a three-dimensional water content map.

In an alternative embodiment, the present disclosure can be used to continuous monitoring of plant water content as it is possible to repeat previous scans to obtain data over time.

Typically, measurements can be obtained at multiple times per second, which enables sampling rates of for example 1000 measurements per metre of vehicle displacement, such that the horizontal (in the direction of displacement) sampling points per metre can be larger or even much larger than the vertical (along the distribution of antenna emitter/receiver pairs) sampling points per metre. As a consequence, the horizontal image resolution can be larger or much larger than the vertical image resolution. Advantageously, this data can be used by computer methods to produce more detailed data on plant growth.

In an embodiment, fruit content can also be measured as fruits contain water. In a further embodiment, fruit water content can be also discriminated from leaf water content as fruits will cause much larger signal attenuation. In another further embodiment, fruit water content can be also discriminated from leaf water content as a vehicle applies a phytosanitary treatment by taking two consecutive measurements and using the fact that leaves will cause differential measurements reflecting the water content of the treatment applied whilst fruit will cause the signal to reach a maximum signal threshold and thus no differential measurement.

The following pertains to experimental results. The laboratory tests carried out to evaluate the method and device disclosed herein used two high gain antennas mounted on a claw. This claw was placed on the end of a robotic arm, which allowed for the scanning of the object being measured. The pair of high gain antennas was connected to a vector network analyzer (VNA) by coaxial cables to measure the S-parameters of the transmitted signal, more precisely, the $S_{12}$ parameter. The model of the VNA was the E8363B PNA Series Network Analyzer (10 MHz to 40 GHz) from Agilent Technologies and the model of the coaxial cables were the KMM24 from Thorlabs and the KBL-2FT-PHS+ from Minicircuits, both prepared for the high frequencies in use during the tests.

For each test, a test element was placed between the emitter/receiver pair to be scanned. The area of this scanning process was defined in the Matlab script with the desired step size. The maximum value of this step size was 3 mm, dictated by the maximum precision of the robotic arm. The maximum size of the scanning area was a 20 cm by 20 cm square, due to physical limitations of the robotic arm. The test sequence consisted of acquiring the $S_{12}$ parameter measured by the VNA on the initial point, send a request to move the robotic arm to the next position, acquire the $S_{12}$ parameter of that position and so on until all the positions defined by the predetermined area for the scanning process were achieved.

Water content measurements were carried out in different objects, plants, and leaves. The size of the objects and plants was limited by the distance between the emitter/receiver pair. This distance was dictated by the size of the two claws used. With the first claw, the distance between antennas was 20 cm, whereas, with the second claw, this distance was 9 cm. Initially, the tests were performed using the first claw, although the distance between the antennas meant that sometimes the VNA measured really low values, in the range of -70 dB to -80 dB for the $S_{12}$ parameter. This value was at the end of the VNA measuring range, and for that reason, the second claw was created to improve the tests performance and to increase the range of intensity values acquired by the VNA.

The different tests performed with plants and leaves will be presented in the following paragraphs. The leaves used during the tests were suspended from a wooden structure so that the only object in the middle of the emitter/receiver pair was the leaves. The same type of leaf was used throughout the tests to decrease the number of variables. As for the plant, its size was small enough to be fitted between the antennas and to allow for the scanning procedure.

A test using cactus-like plant was performed, as presented in **Fig. 6.** This plant was chosen due to its high content in water, causing a greater attenuation on the intensity of the signal. The analysis of the collected data for this test confirms that the signal is strongly attenuated when the signal is passing through the plant. This is perceptible by analyzing the images obtained from the collected data, where the brightest parts of the images correspond to areas where a leaf of the plant is directly in the middle of the emitter/receiver pair, and the darkest areas correspond to the void spaces between each leaf, as observed in **Fig. 6.** The scanning area of this test was a rectangle of 20 cm by 15 cm with a step size of 5 mm, corresponding to 1200 different points. The results are from three different frequencies (19.5 GHz, 19.75 GHz and 20 GHz) as well as the average of the attenuation in the range of 19.5 to 20 GHz. The analysis of the resulting images reveals better results using the average of the frequency range.

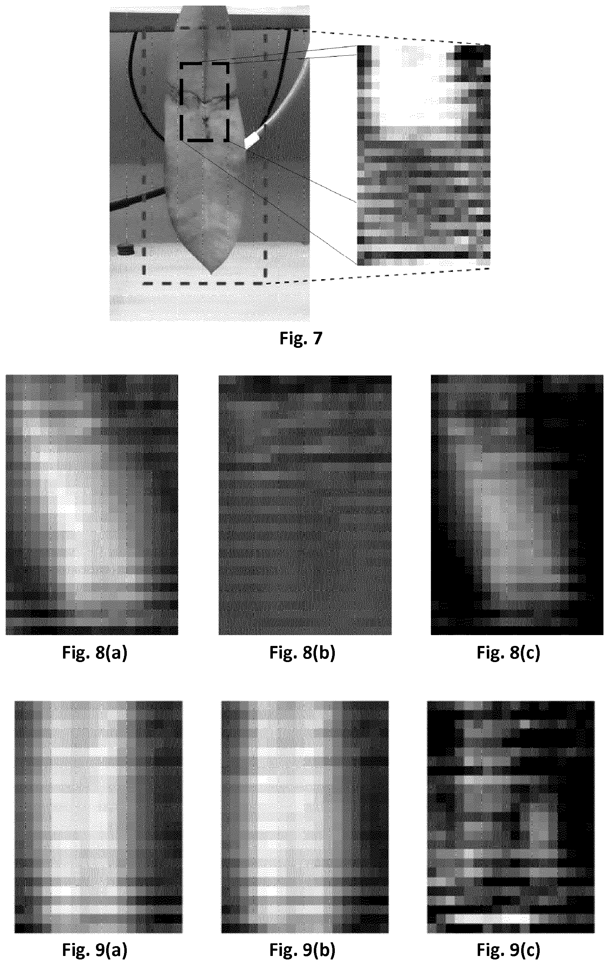

The method and device disclosed herein is able to scan and measure in detail the water content of object of interest with regions with different hydration levels. The disclosure was tested using a leaf with a green region (upper, leaf petiole) and a dry region (bottom, leaf apex) as illustrated on the left side of **Fig. 7.** The leaf was directly extracted from the tree in this condition, with almost half of the leaf in a dry state. The scanning area of this test was a rectangle of 12 cm by 7 cm with a step size of 0.4 cm, corresponding to 540 different points. As observed in **Fig. 7,** the disclosed invention measured higher water content and further presented brighter pixels in the upper region of the generated 2D image, which corresponds to the green region observed near to the leaf petiole. Conversely, the middle and bottom part of the generated image comprised darker pixels, which resulted from the reduced signal attenuation suffered by the microwaves transmitted along this dry region of the leaf near the apex that was measured by the disclosure. This correlates to the real image since the brown portion of the leaf has less water content in comparison to the green part.

The disclosure is also able to measure water content along time. This test consisted of starting with a green leaf freshly taken from a tree, measure its mass using a high precision scale and perform a scanning test. Next, the leaf was left to dry naturally for two days, before being again weighted and scanned. For this test, the size of the scanning area was a 12 cm by 8 cm rectangle with a step size of 0.4 cm, corresponding to a total of 600 different points.

The weight of the leaf decreased between each scanning procedure, starting at 1.1094 grams and finishing at 0.3908 grams. This loss of weight by the leaf is mainly due to the evaporation of water during the natural drying process, since the physical structure of the leaf stayed intact during the drying process and the scanning procedures. This loss of weight corresponds to a loss of water by the leaf, thus affecting each test scan results. The results of this analysis are shown in **Fig. 8,** where **Fig. 8a** represents the result of the scan of the green leaf (with a mass of 1.1094 grams), **Fig. 8b** represents the result of the dry leaf (with a mass of 0.3908 grams) and image **Fig. 8c** is the difference between the results of the green leaf and the dry leaf. The images were obtained by normalizing both matrices with the global maximum and minimum between the matrices acquired from both scanning procedures. The fact that the green leaf result has a brighter area in the zone of the leaf, whereas, on the dry leaf the result is an image almost black and uniform, indicates a clear sensibility of the system to the water content inside the leaf. The lack of a clear contrast between the dry leaf and the area of the image without the leaf in **Fig. 8b** indicates that there is not a significant attenuation of the signal due to the lack of water on the dry leaf.

The analysis of the sum of all the elements of the normalized matrices also gives a clear indication of the effects of the water content of the leaf on the signals' attenuation. The sum of the values of the normalized matrix of the green leaf was 262.20, whereas, for the dry leaf this sum was 156.47. This difference is explained due to the fact that a greater attenuation of the signal is represented by a number closer to 1 on the matrix, thus giving a bigger sum of the elements of the matrix of the green leaf.

The analysis of the difference between the maximum and minimum values recorded by the VNA also reveals some information about the leaves water content. For the data recorded for the green leaf, the difference between the maximum and the minimum values was 6.59 dB, whereas, on the dry leaf, this value was 2.57 dB. This difference of 4.02 dB between these two values reveals a clear indication of a greater effect on the signals' attenuation with the green leaf.

The same test was repeated using a smaller distance between antennas, this time with the scanning procedure being repeated six times over the course of two days and the mass of the leaf being measured before each scan. The values of the leaves mass for each scanning procedure are shown in **Table 1,** with the first three scans being performed on the first day and the following scans on the second day.

Table 1 - Leaves mass at each scan (test) and corresponding difference between the maximum and minimum values of the matrices of each test result.

| | Day 1 | | | Day 2 | | |
|---|---|---|---|---|---|---|
| | Test 1 | Test 2 | Test 3 | Test 4 | Test 5 | Test 6 |
| **Mass (g)** | 1.0472 | 0.9741 | 0.9336 | 0.6181 | 0.5652 | 0.5311 |
| **Difference Max - Min (dB)** | 3.87 | 3.74 | 3.73 | 1.92 | 1.86 | 1.58 |

The analysis of the difference between the maximum and minimum values of the data collected for each test reveals an indication of the effect of the leaves water content on the signal attenuation. The higher the value of the leaves mass (corresponding to a higher water content), the higher the attenuation on the signals' intensity, represented by a greater difference between the maximum and minimum values of the of the tests performed on the first day in relation to the tests on the second day, as shown in **Table 1.**

Reference is made to **Fig. 9** to disclose the results on water sensing after applying a water-based agricultural treatment. The purpose of this test was to check the sensitivity of the disclosure to small changes in the quantity and position of water on a green leaf. The scanning area of this test was a 10 cm by 7 cm rectangle with a step size of 0.4 cm, corresponding to 450 different points. Water content was measured at two different timepoints - the first immediately after water spraying (Fig. 9a) and the second, two hours and half later (Fig. 9b). Despite the small visual difference between **Fig. 9a** and **Fig. 9b,** the resulting difference of these two images shows some bright pixels in the leaf apex that is shown in the lower part of the image **(Fig. 9c).** These pixels correspond to a leaf region where the signal attenuation due to the presence of water in the leaf is higher. These bright pixels are located in the leaf apex (lower part of **Fig. 9c)** as expected since gravity pulls down the drops of water towards the apex. The fact that the test took about two and a half hours to be completed was also a factor to this result as some of the water on the top part of the leaf evaporated before the scan passed over that part of the leaf. The analysis of the pixel values of each image for the two timepoints also revealed that there is a bigger sum for the matrix of the leaf with water on its surface, which again confirms the effect of water on the signals' attenuation. The sum of the values of this matrix was 242.453, whereas, for the matrix of the values for the leaf without water, this sum corresponds to 230.723.

Preferably, for avoiding parallax measurement errors, the beam is unfocused, propagating in constant width, i.e. constant spatial beam profile - from each emitter, through the respective vegetation location to be measured, to the respective receiver (i.e. without concentration or spreading of the beam).

When using a vehicle, the horizontal resolution may be variable, for example taking into account the precision of the geolocation or the displacement sensor. Thus, the horizontal and vertical resolution may differ when using a vehicle.

The term "comprising" whenever used in this document is intended to indicate the presence of stated features, integers, steps, components, but not to preclude the presence or addition of one or more other features, integers, steps, components or groups thereof.

It is to be appreciated that certain embodiments of the disclosure as described herein may be incorporated as code (e.g., a software algorithm or program) residing in firmware and/or on computer useable medium having control logic for enabling execution on a computer system having a computer processor, such as any of the servers described herein. Such a computer system typically includes memory storage configured to provide output from execution of the code which configures a processor in accordance with the execution. The code can be arranged as firmware or software to configure the machine in which it is executed to perform the associated functions, as described herein.

The disclosure should not be seen in any way restricted to the embodiments described and a person with ordinary skill in the art will foresee many possibilities to modifications thereof. The above described embodiments are combinable. The following claims further set out particular embodiments of the disclosure.

**Claims**

1. Device for measuring plant water present in vegetation comprising:

    a microwave emitter of a beam, wherein the beam is displaceable to pass through each of a plurality of locations of the vegetation;
    a microwave receiver;
    an electronic data processor connected to receive a signal from the microwave receiver and to control the displacement of said beam;
    wherein the microwave emitter is arranged to direct said displaceable beam towards the microwave receiver for measuring attenuation of the beam passing through each of said plurality of locations of the vegetation;
    wherein the electronic data processor is configured for calculating the attenuation of the displaceable beam between the emitter and receiver to obtain a 2D image corresponding to the measurement of water present in the vegetation.

2. Device for measuring plant water present in vegetation according to the previous claim wherein the microwave receiver comprises a plurality of individual receivers.

3. Device for measuring plant water present in vegetation according to the previous claim wherein the microwave receiver comprises a plurality of individual microwave receivers linearly arranged and distributed, such that each receiver receives the beam passing through each of said plurality of locations of the vegetation, in particular the plurality of individual microwave receivers being linearly arranged and distributed along a vertical direction.

4. Device for measuring plant water present in vegetation according to any of the claims 2-3 wherein the microwave emitter is arranged to direct said displaceable beam towards each of said individual microwave receivers.

5. Device for measuring plant water present in vegetation according to the previous claim wherein the microwave emitter is arranged to direct said displaceable beam towards one by one of said individual microwave receivers, such that each receiver receives the beam passing through each of said plurality of locations of the vegetation.

6. Device for measuring plant water present in vegetation according to any of the claims 2-5 wherein the microwave emitter comprises a steerable beam antenna, in particular a steerable beam phase-shift antenna, for directing said microwave beam between each of said individual microwave receivers.

7. Device for measuring plant water present in vegetation according to any of the claims 2-6 wherein the microwave emitter comprises a plurality of individual emitters which are individually switchable for directing said microwave beam between each of said individual microwave receivers.

8. Device for measuring plant water present in vegetation according to the previous claim wherein the microwave emitter comprises a plurality of individual microwave emitters linearly arranged and distributed such that each receiver receives the beam emitted from each of said emitters passing through each of said plurality of locations of the vegetation, in particular the plurality of individual microwave emitters being linearly arranged and distributed along a vertical direction.

9. Device for measuring plant water present in vegetation according to any of the previous claims wherein said beam is displaceable by the device being movable along the vegetation to be measured in particular along a horizontal direction, in particular the microwave emitter and microwave receiver being jointly mounted in the device, further in particular the microwave emitter and microwave receiver being jointly mounted in opposite ends of an arched structure.

10. Device for measuring plant water present in vegetation according to the previous claim wherein the device is a vehicle and the microwave emitter and microwave receiver are mounted on the vehicle for moving along the vegetation such that the beam is displaceable to pass through each of the plurality of locations of the vegetation as the vehicle moves.

11. Device for measuring plant water present in vegetation according to the previous claim comprising a satellite geolocation sensor, a radiofrequency geolocation sensor or a displacement sensor for synchronizing the measurement of water present in the vegetation with the device location determining which the plurality of locations of the vegetation is being measured as the vehicle moves.

12. Device for measuring plant water present in vegetation according to the previous claim comprising a second microwave emitter and a second microwave receiver mounted on the vehicle for moving along the vegetation, such that the second emitter-receiver pair measure attenuation of the beam passing through each of a plurality of locations of the vegetation after the first emitter-receiver pair have measured attenuation of the beam passing through each of the same plurality of locations of the vegetation.

13. Device for measuring plant water present in vegetation according to the previous claim, wherein the electronic data processor is configured for calculating the difference in attenuation of the displaceable beam between the first emitter-receiver pair and the second emitter-receiver pair to obtain a 2D image corresponding to the difference in the measurement of water present in the vegetation.

14. Device for applying a water-based agricultural treatment to vegetation according to any of the previous claims comprising a dispenser for applying the treatment and being arranged to:

   measure the water present in the vegetation before applying the treatment and adjust the amount of treatment applied in respect of the measured water; and/or
   measure the water present in the vegetation before and after applying the treatment for calculating the difference in the measurement of water present in the vegetation to measure the applied treatment.

15. Method of operation of a device for measuring plant water present in vegetation, said device comprising a microwave emitter of a beam displaceable to pass through each of a plurality of locations of the vegetation; a microwave receiver; an electronic data processor connected to receive a signal from the microwave receiver and to control the displacement of said beam; said method comprising:

   moving said displaceable beam by the microwave emitter towards the microwave receiver for measuring attenuation of the beam passing through each of a plurality of locations of the vegetation;
   calculating, by the electronic data processor, the attenuation of the displaceable beam between the emitter and receiver to obtain a 2D image corresponding to the measurement of water present in the vegetation.

Fig. 1

Fig. 2A

Fig. 2B

**Fig. 3**

**Fig. 4**

**Fig. 5**

**Fig. 6**

**Fig. 7**

**Fig. 8(a)**

**Fig. 8(b)**

**Fig. 8(c)**

**Fig. 9(a)**

**Fig. 9(b)**

**Fig. 9(c)**

Europäisches
Patentamt

European
Patent Office

Office européen
des brevets

# EUROPEAN SEARCH REPORT

Application Number

EP 19 16 6018

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | OGAWA Y ET AL: "Monitoring of water/ice state using millimeter waves for the agricultural field", INFRARED AND MILLIMETER WAVES, 2004 AND 12TH INTERNATIONAL CONFERENCE ON TERAHERTZ ELECTRONICS, 2004. CONFERENCE DIGEST OF THE 2004 JOINT 29 TH INTERNATIONAL CONFERENCE ON KARLSRUHE, GERMANY SEPT. 27 - OCT. 1, 2004, PISCATAWAY, NJ, USA,IEEE, 27 September 2004 (2004-09-27), pages 451-452, XP010795056, ISBN: 978-0-7803-8490-3 * page 452; figure 3 * * abstract * | 1-15 | INV. G01N33/00 G01N22/04 A01G25/16 |
| X | DOMINGO SANCHO-KNAPIK ET AL: "Microwave-band (1730MHz) accurately estimates the relative water content in poplar leaves. A comparison with a near infrared water index (/)", AGRICULTURAL AND FOREST METEOROLOGY, ELSEVIER, AMSTERDAM, NL, vol. 151, no. 7, 27 January 2011 (2011-01-27), pages 827-832, XP028384106, ISSN: 0168-1923, DOI: 10.1016/J.AGRFORMET.2011.01.016 [retrieved on 2011-02-03] * paragraphs [02.1], [02.2] * * page 831 * | 1,9,10, 15 | |

-----

-----

-/--

TECHNICAL FIELDS
SEARCHED     (IPC)

G01N
A01G

The present search report has been drawn up for all claims

| Place of search | Date of completion of the search | Examiner |
|---|---|---|
| The Hague | 18 October 2019 | Kraus, Leonie |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

.....................................................................
& : member of the same patent family, corresponding
    document

EPO FORM 1503 03.82 (P04C01)

1

Europäisches
Patentamt

European
Patent Office

Office européen
des brevets

# EUROPEAN SEARCH REPORT

Application Number

EP 19 16 6018

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | QUEMADA C ET AL: "A Real-Time Non-Destructive Water Status Monitoring System at Terahertz Band", 2018 15TH EUROPEAN RADAR CONFERENCE (EURAD), EUROPEAN MICROWAVE ASSOCIATION, 26 September 2018 (2018-09-26), pages 465-468, XP033453464, DOI: 10.23919/EURAD.2018.8546613 [retrieved on 2018-11-26] * the whole document * | 1-15 | |
| A | ES 2 385 942 A1 (CT DE INVESTIGACION Y TECNOLOGIA AGROALIMENTARIA DE ARAGON CITA [ES]) 3 August 2012 (2012-08-03) * abstract; claims * | 1-15 | |

TECHNICAL FIELDS
SEARCHED    (IPC)

The present search report has been drawn up for all claims

| Place of search | Date of completion of the search | Examiner |
|---|---|---|
| The Hague | 18 October 2019 | Kraus, Leonie |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 2 of 2

## ANNEX TO THE EUROPEAN SEARCH REPORT
## ON EUROPEAN PATENT APPLICATION NO.

EP 19 16 6018

This annex lists the patent family members relating to the patent documents cited in the above-mentioned European search report.
The members are as contained in the European Patent Office EDP file on
The European Patent Office is in no way liable for these particulars which are merely given for the purpose of information.

18-10-2019

| Patent document cited in search report | | Publication date | Patent family member(s) | Publication date |
|---|---|---|---|---|
| ES 2385942 | A1 | 03-08-2012 | NONE | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- KR 101824652

**Non-patent literature cited in the description**

- **E.RAYMOND HUNT ; BARRETT N ROCK.** Detection of changes in leaf water content using near- and middle-infrared reflectances. *Remote Sensing of Environment,* 1989, vol. 30 (1), 43-54

- **G. SCHIAVON ; D. SOLIMINI ; A. BURINI.** Sensitivity of multi-temporal high resolution polarimetric C and L-band SAR to grapes in vineyards. *2007 IEEE International Geoscience and Remote Sensing Symposium,* July 2007, 3651-3654